Europäisches Patentamt

European Patent Office

Office européen des brevets

(11) Publication number: **0 059 052**
**B1**

(12) **EUROPEAN PATENT SPECIFICATION**

(45) Date of publication of patent specification: **07.08.85**

(21) Application number: **82300734.9**

(22) Date of filing: **15.02.82**

(51) Int. Cl.⁴: **C 07 D 309/36, C 07 D 309/38**

(54) **Process for the preparation of a substituted pyrone.**

(30) Priority: **20.02.81 US 236352**

(43) Date of publication of application:
**01.09.82 Bulletin 82/35**

(45) Publication of the grant of the patent:
**07.08.85 Bulletin 85/32**

(84) Designated Contracting States:
**BE CH DE FR GB IT LI NL**

(56) References cited:
**US-A-3 657 426**

**BERICHTE DER DEUTSCHEN CHEMISCHEN
GESELLSCHAFT, vol.65, 1932, no.161, Berlin
(DE), W.BORSCHE et al.:"Untersuchungen über
die Bestandteile der Kawawurzel,
XII.Mitteil.:Über Yangonalacton und
Triacetsäure", pages 820-828**

(73) Proprietor: **Rohm and Haas Company
Independence Mall West
Philadelphia, Pennsylvania 19105 (US)**

(72) Inventor: **Opie, Thomas Ranson
345 Park Avenue
Collingswood New Jersey 08108 (US)**

(74) Representative: **Wood, Peter Worsley Spencer
et al
Rohm and Haas Company Patent Department
Chesterfield House Bloomsbury Way
London WC1A 2TP (GB)**

## Description

This invention concerns a process for the preparation of a substituted pyrone namely 4 - hydroxy - 6 - methyl - 2 - pyrone.

The compound 4 - hydroxy - 6 - methyl - 2 - pyrone (HMEP) has been used, *inter alia*, as an intermediate for the preparation of 1 - (4 - chlorophenyl) - 1,4 - dihydro - 6 - methyl - 4 - oxo - 3 - pyridazine carboxylates and related plant growth regulating agents (see DE—A—2,808,795).

HMEP has been prepared, *inter alia*, by deacetylation of dehydroacetic acid (DHAA) in hot 90% sulfuric acid, see W. Borsche and B. K. Blount, *Berichte der Deutschen Chemischen Gesellschaft, 65*, 827 (1932), J. N. Collie. *J. Chem. Soc., 59*, 607—609 (1891) and US—A—3,657,426.

The acidic deacetylation conditions using $H_2SO_4$ are critical since heating DHAA in 85% sulfuric acid produces primarily the rearrangement product 2,6 - dimethyl - 4 - pyrone - 3 - carboxylic acid; see J. N. Collie and T. P. Hilditch, *J. Chem. Soc., 91*, 787 (1907).

The preparation of HMEP from DHAA in sulfuric acid as disclosed by J. N. Collie, is described in more detail by Borsche and Blount. This Collie-Borsche-Blount procedure is not suitable for batch production of HMEP because of the enormous rates of heat transfer that would be required. It or any other procedure requiring such large ratios of $H_2SO_4$ (3 to 1) and quench water (4 to 1) to DHAA suffers from problems of HMEP losses in the quench mixture, large volumes of acidic waste to be treated or recycled, and low kettle productivity. Moreover, the procedures are run under conditions (ca. two minutes at 130°C. or an unspecified time at 120°C.) that cannot readily be duplicated or controlled on a commercial scale.

This invention provides a process for the preparation of HMEP which is more advantageous than the known procedures for deacetylating DHAA using sulfuric acid.

The process of the invention comprises reacting dehydroacetic acid (DHAA) with aqueous $H_2SO_4$ containing 91—99% by weight of $H_2SO_4$ at a temperature from 60°C. to 140°C. under a pressure of $1.33 \times 10^{-2}$ bar (10 millimetres of mercury absolute) to 5.16 bar (388 centimetres of mercury absolute), characterised in that the concentration of $H_2SO_4$ in the aqueous $H_2SO_4$ is 91—99% by weight and (A) the $H_2SO_4$ plus $H_2O$:DHAA weight ratio is from 1.41 to 3:1 and (B) the DHAA is initially reacted with from 94% to 99% $H_2SO_4$ followed by one or more staged $H_2O$ additions during the reaction whereby the total weight of $H_2O$ added and initially present in the $H_2SO_4$ charged is 10% or less of the weight of $H_2SO_4$ (100% basis) initially charged, said staged $H_2O$ addition(s) being optional when up to 96% $H_2SO_4$ is initially charged.

It is preferred in the process of this invention to carry out one or more staged water addition. A preferred temperature range for the process of this invention is from about 80° to about 120°C.

The process of the present invention can be carried out either as a batch process or as a continuous process. In a more preferred process of the present invention the total weight of water added in staged increments and present initially in the $H_2SO_4$ is from 4% to 7% of the weight of the sulfuric acid (100% basis) initially charged. Preferred reaction times are from 10 minutes to 15 hours. The preferred reaction pressure is 1.01 bar (760 millimitres of mercury absolute).

The advantage of the process of the invention over the prior art is that in the process of the present invention the water content of the sulfuric acid utilized in the HMEP preparation is controlled so that the sulfuric acid concentration is between 91% and 99%, preferably about 94—96%, more preferably 96—98% $H_2SO_4$ with about 2—3% by weight of water added in staged increments. The advantages obtainable by the process include:

1. The possibility of using lower minimum $H_2SO_4$/DHAA charge ratios resulting in reduced waste volumes and higher productivity.

Using staged water additions and an initial charge of 1.4—2.2 parts by weight of 96.9—97.5% $H_2SO_4$ per part by weight of DHAA, the process can provide isolated HMEP yields of 71—80% after 2.1—2.5 hours at 100°C. The charge ratios can represent 47%—73% of the sulfuric acid charge utilized in the Collie-Borsche-Blount procedure. This improvement further permits 25%—38% reductions in the weight of quench water utilized as well. The overall result is a large reduction in the volume of acidic waste produced and a large increase in kettle productivities.

2. Increased yield and selectivity over those obtained with 90% sulfuric acid initially charged, especially at sulfuric acid/DHAA weight ratios of 2.5 or less:1.

The isolated HMEP yields of 71—80% discussed above with staged water additions and an isolated HMEP yield of 75% obtained using 94% $H_2SO_4$ and DHAA in a 2:1 weight ratio are higher than most of the yields reported using the Collie-Borsche-Blount procedure. In addition, they are much higher than the isolated HMEP yields obtained using 90% sulfuric acid at 100°C. with lower sulfuric acid/DHAA weight ratios. For example, use of 90% sulfuric acid at a 2.37:1 aqueous $H_2SO_4$/DHAA ratio gave only a 60.5% isolated A. I. yield of HMEP while at a 1.75:1 weight ratio, the isolated HMEP yield was only 31.8%. Hence the use of the drier sulfuric acid and/or staged water addition(s) in the process of the invention greatly improves the yield and selectivity of HMEP production at sulfuric acid/DHAA weight ratios of 2.5 or less:1.0.

3. Ready applicability to batch or continuous processing.

The lower reaction temperature and improved control of the water content of the sulfuric acid in this invention gives a process which is applicable to batch or continuous processing. Several 1136 l (300-gallon) (scale) batches of HMEP have been conducted using a preferred version of the invention (see Example 10 appearing later).

The present invention consists of controlling the water content of the sulfuric acid so that the sulfuric acid concentration is between 91% and 99%, preferably about 94%—96%, more preferably 96—98% when 2—3% of water is added in staged increments as the reaction proceeds. In the process of the invention the reaction temperature may be reduced and heating and cooling cycles may be lengthened to permit a more readily controlled deacetylation. The invention is based upon the discovery that keeping the acid initially charged drier than the 90% sulfuric acid utilized in the prior art permits a more selective, practical preparation of HMEP to occur at lower DHAA/sulfuric acid ratios. The preparation is especially selective if the reaction is initiated with relatively dry (ca. 97% or more) sulfuric acid and small incremental portions of water are added during the deacetylation to bring the total water content up to the equivalent of starting with 93—95% sulfuric acid.

If, however, the acid is too dry (e.g. 97%—99% sulfuric acid) with no incremental water additions, the deacetylation stalls and much DHAA remains uncoverted, especially when the $H_2SO_4$ /DHAA weight ratio is less than about 2.5 to 1.0. On the other hand, when the acid initially charged is too wet (e.g., the 90% sulfuric acid utilized in the prior art), the yield of the by-products 2,6 - dimethyl - 4 - pyrone - 3 - carboxylic acid (DMPCA) and 2,6 - dimethyl - 4 - pyrone (DMP) increases, especially if the $H_2SO_4$ /DHAA weight ratio is less than about 2.5:1.0.

The following examples 1—5, 7 and 10 are provided to illustrate the process of the present invention. Examples 6, 8 and 9 are presented by way of comparison.

All experiments except Examples 1, 2 and 10 were conducted in the laboratory using a stoppered 300 ml. three-neck, round-bottom flask equipped with a mechanical paddle stirrer and a thermometer. The flask was heated by a heating mantle controlled by an Instruments for Research and Industry Therm-o-Watch controller. Examples 1 and 10 were run in 38 litre (10 U.S. gallons) and 1136 litre (300 U.S. gallons) glass-lined kettles, respectively, and heated with steam or hot water. In all cases except Examples 1 and 10, the dehydroacetic acid was charged before the sulfuric acid, but the order of addition is immaterial to the invention.

Quenches of the reaction mixtures were conducted using 1.39 or 1.77 parts by weight of quench water per part weight of sulfuric acid (100% basis) used. The decrease in isolated yields using the lower quench ratio was only 0.6%. The chemical yields of DHAA, HMEP, DMPCA and DMP in the unquenched reaction mixtures were determined by NMR spectroscopy of the (ring) vinyl peaks at δ5.5—7.5.

Example 1

6.67 Kg (14.7 lbs) of dehydroacetic acid and 13.24 Kg (29.2 lbs) of 96.9% sulfuric acid were charged to an unpressurized glass-lined 38 l (10 U.S. gallon) kettle and heated with stirring over 65 minutes to 98°C. Then the mixture was held for 2.5 hours at 96—98°C. Two 14.2 g (five-ounce) tap water charges were added after 30 and 60 minutes of the hold period had elapsed; during the hold period, the color of the reaction mixture changed from a golden brown to a reddish brown. 28 g (one-ounce) samples were taken for NMR analysis after one hour and two hours of the hold period had elapsed.

After the 2.5 hour hold, the agitated reaction mixture was cooled to 22°C. over 65 minutes. Then 22.8 Kg (50.2 lbs) of tap water were added to the reaction mixture over 105 minutes at 22—46°C.; a finely divided white slurry of HMEP precipitated. The HMEP slurry was cooled to 10°C. and filtered onto a chock filter equipped with a cheesecloth. The wet cake was washed with two 10 Kg (22.0 lbs) portions of tap water and sucked dry to give 7.58 Kg (16.7 lbs) of 49.7% solids wet HMEP. The crude isolated yield of HMEP was 3.76 Kg (8.3 lbs) or 75.6%; the purity of a sample of the HMEP was 98—100% (four determinations) by TLC densitometry. NMR analysis of the two-hour sample indicated a 78.5% reaction yield of HMEP and a 14.5% yield of DMPCA with 7.0% uncoverted DHAA.

Example 2

111.3 g of DHAA and 220.4 g of 96.9% $H_2SO_4$ were charged to a 1,000-ml., 4-necked round bottomed flask equipped with an addition funnel, paddle stirrer and thermometer. The stirred mixture was rapidly heated to 110°C. and held for 5 hours at 109—112°C. After 3 hours, a 1.807 g water charge was added, after 4.27 hours, a second 1.800 g water charge was added. Four NMR samples removed during the hold period gave the following analysis:

| Time | % DHAA | % HMEP | % DMPCA |
|---|---|---|---|
| 45 min. | 34.5 | 54.5 | 11.0 |
| 2.5 hr. | 26.9 | 57.2 | 15.9 |
| 3.75 hr. | 7.5 | 74.8 | 17.7 |
| 5.0 hr. | 2.7 | 79.5 | 18.8 |

3

At the end of the hold period, the mixture was cooled and quenched with 380 g of tap water at 20.0—52.5°C. over 28 minutes. The resulting white HMEP slurry was cooled to 0—5°C., held for one hour and suction filtered to give a HMEP wet cake. The HMEP was washed twice with 165 g portions of tap water and vacuum dried to give 62.4 g of 95.4% pure dry HMEP. The corrected HMEP yield was 72%.

Examples 3—5 and 10 in Table 1 give further documentation of the use of staged water additions and low $H_2SO_4$/DHAA ratios under the claims of this invention.

Example 7 in Table 1 illustrates the preparation of HMEP under this invention using a 2.0/1.0 94% $H_2SO_4$/DHAA weight ratio.

Comparative Examples 6, 8 and 9 in Table 1 illustrate the preparation of HMEP at lower temperatures and lower sulfuric acid ratios using in Examples 8 and 9 the 90% sulfuric acid specified by the prior art. The yields of HMEP are clearly lower than those obtained using similar $H_2SO_4$/DHAA ratios under the drier conditions and/or staged water addition(s) used in the process of this invention.

Example 10 illustrates the large scale preparation of HMEP using one of the preferred sets of conditions of this invention.

The results of these experiments are presented in Table II below. In Table II a dash indicates "not determined".

TABLE I
Preparations of HMEP by deacetylation of DHAA

Reaction conditions

| Example | Time | Temp. °C. | Weight of aqueous $H_2SO_4$ | Weight DHAA | Init. conc. of $H_2SO_4$ | Water charges and times after start of hold period |
|---|---|---|---|---|---|---|
| 1 | 2.5 hr. | 96—98 | 13.24 Kg | .6.67 Kg | 96.9% | 142 g., 30 min. 142 g., 60 min. |
| 2 | 5.0 hr. | 109—112 | 220.4 g. | 111.3 g. | 96.9 | 1.81 g., 3 hr. 1.80 g., 4.27 hr. |
| 3 | 1.0 hr. | 98—105 | 110.5 g. | 50.4 g. | 97.1 | 1.01 g., 30 min. |
| 4 | 2.0 hr. | 98—105 | 81.5 | 50.43 g. | 97.5 | 0.94 g., 30 min. 0.95 g., 60 min. |
| 5 | 2.5 hr. | 96—103 | 71.1 g. | 50.4 g. | 96.9% | 0.96 g., 21 min. 0.77 g., 60 min. 0.81 g., 105 min. |
| 6 | 3.1 hr. | 98—103 | 52.9 g. [a] | 50.45 g. | 99.1 | 0.72 g., 30 min. 0.68 g., 62 min. 0.29 g., 122 min. |
| 7 | 2.1 hr. | 99—106 | 103.0 g.[a] | 50.4 g. | 94.0 | none |
| 8 | 2.0 hr. | 99—105 | 119.2 g.[a] | 50.4 g. | 90.0 | none |
| 9 | 2.0 hr. | 99—105 | 97.9 g.[a] | 55.95 g. | 90.0 | none |
| 10 | 3.65 hr. | 98—111 | 446 Kg[a] | 225 Kg | 97.1 | 6.12 Kg. 30 min. 6.12 Kg. 60 min. |

[a] Actual charges were as follows: Example 4, 76.7 g., 97.1% $H_2SO_4$ and 4.80 g., 104.1% $H_2SO_4$; Example 6, 37.85 g. of 97.1% $H_2SO_4$ and 15.05 g. of 104.1% $H_2SO_4$; Example 7, 100.8 g. of 96.0% $H_2SO_4$ and 2.2 g. of water; Example 8, 111.8 g. of 96.0% $H_2SO_4$ and 7.4 of water; Example 9, 98.8 g. of 96.0% $H_2SO_4$ and 6.1 g. of water; and Example 10, 227 Kg. of 99.1% of $H_2SO_4$, 216 Kg. of 96.6% $H_2SO_4$ and 3.54 Kg. of water.

4

TABLE II
Distribution of reaction mixture components

| Example No. | Mole % of reaction mixture components[a] | | | | Analysis of isolated products | | |
|---|---|---|---|---|---|---|---|
| | HMEP | DHAA | DMPCA[b] | DMP[b] | Crude yield | % HMEP | Isolated yield |
| 1 | 78.5 | 7.0 | 19.5 | — | 3.76 Kg. 75.6% | 98 | 74.0% |
| 2 | 79.5 | 2.7 | 18.8 | — | 62.4 g., 75.5% | 95.4 | 72.0% |
| 3 | 91.1 | 0.0 | 8.9 | — | 30.4 g., 80.4% | — | — |
| 4 | 81.0 | 4.3 | 14.7 | — | 28.4 g., 75.0% | | 75.0 |
| 5 | 83.9 | 0.0 | 16.1 | — | 27.8 g., 73.7% | 96.8 | 71.0 |
| 6 | | | | | 20.5 g., 54.2% | | 54.2 |
| 7 | 74.9 | 9.1 | 12.8 | 3.3 | 28.4 g., 75.1% | 101.5 | 75.1 |
| 8 | 69.3 | 0.6 | 25.8 | 4.4 | 23.0 g., 61.0% | 99.2 | 60.5 |
| 9 | 43.9 | 0.0 | 43.5 | 12.6 | 14.3 g., 34.1% | 93.3 | 31.8 |
| 10 | 88.6 | 4.1 | 9.35 | — | 134.3 Kg. 79.5% | 100 | 79.5 |

[a] Mole % of reaction mixture components, determined by nmr analysis.
[b] DMPCA is 2,6 - dimethyl - 4 - pyrone - 3 - carboxylic acid and DMP is 2,6 - dimethyl - 4 - pyrone:

## Claims

1. A process for the preparation of 4 - hydroxy - 6 - methyl - 2 - pyrone which comprises reacting dehydroacetic acid (DHAA) with aqueous $H_2SO_4$ at a temperature from 60°C to 140°C and under a pressure of $1.33 \times 10^{-2}$ bar (10 millimetres of mercury absolute) to 5.16 bar (388 centimetres of mercury absolute), characterised in that the concentration of $H_2SO_4$ in the aqueous $H_2SO_4$ is 91—99% by weight and (A) the $H_2SO_4$ plus $H_2O$:DHAA weight ratio is from 1.41 to 3:1 and (B) the DHAA is initially reacted with from 94% to 99% $H_2SO_4$ followed by one or more staged $H_2O$ additions during the reaction whereby the total weight of $H_2O$ added and initially present in the $H_2SO_4$ charged is 10% or less of the weight of $H_2SO_4$ (100% basis) initially charged, said staged $H_2O$ addition(s) being optional when up to 96% $H_2SO_4$ is initially charged.

2. A process as claimed in Claim 1 wherein one or more of said staged $H_2O$ additions is (are) made.

3. A process as claimed in Claim 1 or 2, wherein the reaction temperature is from 80°C to 120°C.

4. A process as claimed in Claim 2 or 3, wherein the total weight of $H_2O$ added and initially present in the $H_2SO_4$ charged is from 4—7% of the weight of $H_2SO_4$ (100% basis) initially charged.

5. A process as claimed in Claim 4 wherein the reaction time is from 10 minutes to 15 hours and the reaction pressure 1.01 bar 760 millimetres of mercury absolute.

## Patentansprüche

1. Verfahren zur Herstellung von 4-Hydroxy-6-methyl-2-pyron durch die Umsetzung von Dehydroessigsäure (DHAA) mit wäßriger $H_2SO_4$ bei einer Temperatur von 60°C bis 140°C und unter einem Druck von $1,33 \times 10^{-2}$ bar (10 mm Hg absolut) bis 5,16 bar (388 cm Hg absolut), dadurch gekennzeichnet, daß die Konzentration an $H_2SO_4$ in der wäßrigen $H_2SO_4$ 91 bis 99 Gew.-% beträgt und (A) das Gewichtsverhältnis $H_2SO_4$ plus $H_2O$:DHAA 1,41 bis 3:1 und (B) die DHAA anfänglich mit 94 bis 99% $H_2SO_4$ umgesetzt wird, worauf sich eine oder mehrere abgestufte $H_2O$-Zusätze während der Reaktion anschließen, wobei das Gesamtgewicht an zugesetztem $H_2O$ und an anfänglich in der zugeführten $H_2 SO_4$ vorliegendem $H_2O$ 10% oder weniger des Gewichts der $H_2SO_4$ (100 %-Basis), die ursprünglich zugeführt worden ist, beträgt, wobei die abgestufte(n) $H_2O$-Zugabe(n) erfolgt bzw. erfolgen, wenn bis zu 96% $H_2SO_4$ anfänglich zugeführt worden sind.

2. Verfahren nach Anspruch 1, dadurch gekennzeichnet, daß ein oder mehrere der abgestuften $H_2O$-Zugabe(n) erfolgt bzw. erfolgen.

**0 059 052**

3. Verfahren nach Anspruch 1 oder 2, dadurch gekennzeichnet, daß die Reaktionstemperatur 80 bis 120°C beträgt.

4. Verfahren nach Anspruch 2 oder 3, dadurch gekennzeichnet, daß das Gesamtgewicht an anfänglich zugesetztem und ursprünglich in der zugeführten $H_2SO_4$ vorliegendem $H_2O$ 4 bis 7% des Gewichts der $H_2SO_4$ (100 %-Basis) die ursprünglich zugeführt worden ist, beträgt.

5. Verfahren nach Anspruch 4, dadurch gekennzeichnet, daß die Reaktionszeit 10 Minuten bis 15 Stunden und der Reaktionsdruck 1,01 bar (760 mm Hg absolut) betragen.

**Revendications**

1. Procédé pour la préparation de 4-hydroxy-6-méthyl-2-pyrone qui comprend la réaction de l'acide déhydroacétique (DHAA) avec l'$H_2SO_4$ aqueux à une température de 60°C à 140°C sous une pression de $1,33 \times 10^{-2}$ bar (10 millimètres absolus de mercure) à 5,16 bars (388 centimètres absolus de mercure), caractérisé en ce que la concentration de l'$H_2SO_4$ dans l'$H_2SO_4$ aqueux est de 91—99% en poids et (A) le rapport pondéral $H_2SO_4$ plus $H_2O$/DHAA est de 1,41 à 3/1 et (B) on fait réagir initialement le DHAA avec de l'$H_2SO_4$ de 94% à 99% puis on effectue une ou plusieurs additions successives d'$H_2O$ pendant la réaction, si bien que le poids total d'$H_2O$ ajoutée et initialement présente dans l'$H_2SO_4$ chargé, est de 10% ou moins du poids d'$H_2SO_4$ (base 100%) initialement chargé, l'addition ou lesdites additions d'$H_2O$ étant facultatives lorsque de l'$H_2SO_4$ atteignant 96% est initialement chargé.

2. Procédé comme revendiqué dans la revendication 1 dans lequel une ou plusieurs desdites additions successives d'$H_2O$ est (sont) effectuée(s).

3. Procédé comme revendiqué dans la revendication 1 ou 2 dans lequel la température de réaction est de 80°C à 120°C.

4. Procédé comme revendiqué dans la revendication 2 ou 3 dans lequel le poids total d'$H_2O$ ajoutée et initialement présente dans l'$H_2SO_4$ chargé est de 4—7% du poids d'$H_2SO_4$ (base 100%) initialement chargé.

5. Procédé comme revendiqué dans la revendications 4 dans lequel la durée de réaction est de 10 minutes à 15 heures et la pression de réaction est de 1,01 bar (760 millimètres absolus de mercure).

6